# EUROPEAN PATENT SPECIFICATION

(11) **EP 3 714 890 B1**
(45) Date of publication and mention of the grant of the patent: **21.09.2022**
(21) Application number: 19166113.1
(22) Date of filing: 29.03.2019
(51) Int. Cl.: A61K 31/737, A61K 36/03, A61K 38/39

(54) **COMPOSITION COMPRISING A COLLAGEN HYDROLYSATE AND A FUCOIDAN**
ZUSAMMENSETZUNG MIT EINEM KOLLAGENHYDROLYSAT UND EINEM FUCOIDAN
COMPOSITION COMPRENANT UN HYDROLYSAT DE COLLAGÈNE ET UN FUCOÏDANE

(43) Date of publication of application: 30.09.2020
(73) Proprietor: Acten AG, 8866 Ziegelbruecke (CH)
(72) Inventor: RENZO, Francesco, 8872 Weesen (CH)
(74) Representative: Masala, Gian Tomaso

(56) References cited:
- WO-A1-2018/166807
- WO-A2-2007/140022
- Symbiosis Group Symbiosis ET AL: "*Corresponding author email: Randomized Observational Multicenter Study to Assess the Efficacy and Safety 0f the Association of Fortigel (10 Gr) and Fucoidan (100 Mg) in Patients with Gonarthrosis Journal of Rheumatology and Arthritic Diseases Open Access", , 22 November 2016 (2016-11-22), XP055623732, Retrieved from the Internet: URL:http://symbiosisonlinepublishing.com/r heumatology-arthritic-diseases/rheumatolog y-arthritic-diseases10.pdf [retrieved on 2019-09-18]
- Maritech: "HIGH-PURITY FUCOIDAN EXTRACT", , 31 December 2018 (2018-12-31), XP055623361, Retrieved from the Internet: URL:https://www.marinova.com.au/assets/Mar itech_Fucus_vesiculosus_extract.pdf [retrieved on 2019-09-18]

## Description

### FIELD OF THE INVENTION

The invention relates to a composition comprising at least one collagen hydrolysate and at least one fucoidan providing beneficial effects on synovial joints.

### BACKGROUND

Articular cartilage is a thin covering of a highly specialized connective tissue on the ends of bones surfaces that covers synovial joints. Its principal function is to provide a smooth, lubricated surface for articulation and to facilitate the transmission of loads with a low frictional coefficient. Articular cartilage is endowed with elasticity and at the same time of resistance, and it allows the joints to slide protecting them, at the same time, from frictions.

Articular cartilage is devoid of blood vessels, lymphatics, and nerves and is subject to a harsh biomechanical environment. Most important, articular cartilage has a limited capacity for intrinsic healing and repair. In this regard, the preservation and health of articular cartilage are paramount to joint health. With aging, however, the cartilage undergoes degenerative processes. At the same time, intense sports and overweight can be risk factors for early cartilage wear. When the cartilage is damaged, its protection action on joints may be compromised, leading to joints diseases or injuries.

During or after physical activity, furthermore, cartilage elasticity and resistance may be limiting factors for the function of synovial joints, which may impair or limit the performance of athletes or sport lovers.

Products to protect the joints that limit the progression of damages on cartilages that over time can hinder the performance of common activities are commercially available, for instance the one marketed under the name ACTEN, a dietary supplement comprising hydrolyzed collagen and a fucoidan in a weight ratio of 100:1.

### SUMMARY OF INVENTION

The Applicant has noted that, even if products limiting the progression of damages on cartilages are commercially available, it is however still felt the need to provide further products providing beneficial effects on synovial joints either when the cartilages are damaged or when the cartilage metabolism needs to be assisted, for example during physical activity.

An object of the present invention is therefore the provision of a new and improved composition providing beneficial effects on synovial joints.

Therefore, the present invention relates, in a first aspect, to a composition comprising at least one collagen hydrolysate and at least one fucoidan, wherein the weight ratio between the at least collagen hydrolysate and the at least fucoidan is from 85:1 to 15:1.

The Applicant has indeed noted that thanks to the association of collagen hydrolysate and fucoidan in a specific weight ratio, the composition according to the invention provides beneficial therapeutic effects on synovial joints in case of a synovial joint disease or a synovial joint injury in a patient, helping the articular cartilage to protect synovial joints and to facilitate the transmission of loads with a low frictional coefficient, thus mitigating pain and sufferings linked to joints movements, as well as preventive treatment to avoid future problems

Without wishing to be bound to a specific theory, the Applicant believes that the specific weight ratio between the at least collagen hydrolysate and the at least fucoidan allows to effectively facilitate the transport of collagen in synovial joint, so as to enable it to assist the articular cartilage in its regular function.

In a further aspect, the present invention also relates to a process for preparing the composition according to the invention, said process comprising the steps of:
- providing at least one fucoidan;
- adding and mixing at least one collagen hydrolysate in an amount wherein the weight ratio between the at least one collagen hydrolysate and the at least one fucoidan is from 85:1 to 15:1;
- obtaining the composition.

The process according to the present invention allows obtaining, starting from the aforesaid starting products, the composition according to the invention, using a simple and reliable procedure.

In a preferred embodiment, the process according to the invention comprises the steps of:
a. providing at least one fucoidan;
b. adding to and mixing with the at least one fucoidan a first group of components selected from the group consisting of: water, preservative, nutrient, and mixtures thereof, to obtain a first mixture, wherein said step b is preferably carried out at a temperature from 10 °C to 35 °C, preferably from 20 °C to 30 °C, even more preferably from 22 °C to 27 °C;
c. adding to and mixing with the first mixture of step b. at least one collagen hydrolysate in an amount wherein the weight ratio between the at least one collagen hydrolysate and the at least one fucoidan is from 85:1 to 15:1, to obtain a second mixture;
d. maintaining the second mixture under stirring at a temperature from 10 °C to 35 °C, preferably from 20 °C to 30 °C, even more preferably from 22 °C to 27 °C, for a time interval of from 2 hours to 12 hours, more preferably from 4 hours to 10 hours, more preferably from 6 hours to 8 hours;
e. adding to and mixing with the second mixture after step d. a second group of components selected from the group consisting of: water, sweetening agent, thickening agent, flavoring agent, and mixtures thereof, to obtain a third mixture;
f. optionally adjusting the pH of the third mixture after step e. to a value of from 4 to 6, more preferably from 4.2 to 5.8, even more preferably from 4.3 to 5.6, even more preferably from 4.4 to 5.4, even more preferably from 4.5 to 5.2; and
g. obtaining the composition.

The Applicant has indeed noted that, thanks to the combination of these specific steps and process conditions, an intimate and optimal mixing between the at least one collagen hydrolisate and the at least one fucoidan may be obtained, thus favoring the effectiveness of the composition in providing beneficial effects on synovial joints.

In a further aspect, the present invention relates also to the composition according to the invention for use in the treatment of a synovial joint disease or a synovial joint injury in a patient.

At the same time, the Applicant has also noted that the composition according to the invention provides also beneficial non-therapeutic effects to the cartilage metabolism, providing to the same nutrients that may preserve or improve its elasticity and resistance.

In a further aspect, therefore, the present invention relates also to a dietary supplement made of the composition according to the invention.

Thanks to the association of collagen hydrolysate and fucoidan in a specific weight ratio, the dietary supplement according to the invention is indeed suitable to assist or improve the function of synovial joints during or after physical activity in a healthy subject, thus for example assisting the performance of athletes or sport lovers. Assistance and improvements of the function of synovial joints during or after physical activity may reduce the manifestation of fatigue in a healthy subject, or reducing the amount of time needed for removing it.

In a preferred embodiment, therefore, the present invention also relates to the use of the dietary supplement according to the invention, for assisting or improving the function of synovial joints during or after physical activity in a healthy subject.

### BRIEF DESCRIPTION OF THE DRAWINGS

Figure 1 shows the normalized VAS scores obtained from a double blind clinical trial comparing the composition according to Example 1 with intraarticular hyaluronic acid and platelet-rich plasma on patients suffering from osteoarthritis; and
Figure 2 shows the normalized WOMAC scores obtained from a double blind clinical trial comparing the composition according to Example 1 with intraarticular hyaluronic acid and platelet-rich plasma on patients suffering from osteoarthritis;

### DETAILED DESCRIPTION OF THE INVENTION

The present invention relates, in a first aspect, to a composition comprising at least one collagen hydrolysate and at least fucoidan, wherein the weight ratio between the at least one collagen hydrolysate and the at least one fucoidan is from 85:1 to 15:1.

The Applicant has indeed noted that thanks to the association of collagen hydrolysate and fucoidan in a specific weight ratio, the composition according to the invention provides beneficial therapeutic effects on synovial joints in case of a synovial joint disease or a synovial joint injury in a patient, helping the articular cartilage to protect synovial joints and to facilitate the transmission of loads with a low frictional coefficient, thus mitigating pain and sufferings linked to joints movements.

Without wishing to be bound to a specific theory, the Applicant believes that the specific weight ratio between the at least collagen hydrolysate and the at least fucoidan allows the at least one of fucoidan to effectively facilitate the transport of collagen in synovial joint, so as to enable it to assist the articular cartilage in its regular function.

In the context of the present application, the expression "healthy subject" indicates a subject to which a synovial joint disease, that include, but not limited to, Osteoarthritis (OA), chondropathy, synovitis, subchondral bone remodelling and osteophyte formation, or a synovial joint injury has not been diagnosed by radiological and clinical criteria.

Preferably, the weight ratio between the at least one collagen hydrolysate and the at least one fucoidan is from 75:1 to 25:1, more preferably from 65:1 to 30:1, still more preferably from 45:1 to 35:1.

The use of such a weight ratio advantageously leads to more apparent beneficial effects on synovial joints, thanks to the optimal association of collagen hydrolysate and fucoidan.

Preferably, the composition according to the invention comprises, with respect to the total weight of the composition, from 30% to 55% by weight of the at least one collagen hydrolysate, more preferably from 35% to 50% by weight of the at least one collagen hydrolysate, still more preferably 40% to 45% by weight of the at least one collagen hydrolysate.

This amount of collagen hydrolysate has been proved to be optimal for the provision of beneficial effects on synovial joints.

Preferably, in the composition according to the invention the at least one collagen hydrolysate has an average molecular weight in the range of 2,000-5,000 daltons.

Suitable commercially available collagen hydrolysates are commercially available, for instance those markeded under the name Fortigel^{®} from Gelita Art.-No. 218010 or similar.

The composition according to the invention comprises at least one fucoidan.

Fucoidans are sulfated polysaccharides (MW: average 20,000) found mainly in various species of brown algae and brown seaweed such as mozuku, kombu, Fucus vesiculosus (also known as bladder wrack, black tang, rockweed, bladder fucus, sea oak, black tany, cut weed, dyers fucus, red fucus, or rock wrack), wakame, and hijiki. Variant forms of fucoidan have also been found in animal species, including the sea cucumber. There are at least two known distinct forms of fucoidans: F-fucoidan, which is >95% composed of sulfated esters of fucose, and U-fucoidan, which is approximately 20% glucuronic acid.

Preferably, in the composition according to the invention the at least one fucoidan is a Fucus vesiculosus extract, more preferably is a Fucus vesiculosus glyceric extract, advantageously having a concentration of fucoidan 85 w/v.

Suitable fucoidans extracted from Fucus vesiculosus are commercially available, for instance the product Maritech^{®} Fucus vesiculosus extract, CAS: 9072-19-9 or similar.

In addition to the components described above, the composition according to the present invention can advantageously comprise one or more further components preferably selected from the group consisting of: water, preservative, nutrient, sweetening agent, thickening agent, and flavoring agent, and mixtures thereof. Among nutrients, vitamins, macronutrients minerals, trace minerals, and mixtures thereof are preferred.

Among vitamins, vitamin C (L-ascorbic acid) is preferred.

Among trace minerals, zinc is preferred. Suitable forms in which trace minerals may be present in the composition according to the present invention are, for example, salts.

Preferably, the preservative is selected from the group consisting of: potassium sorbate, sodium benzoate, and mixtures thereof. More preferably, in the composition according to the invention the preservative comprises a mixture of potassium sorbate and sodium benzoate.

Among sweetening agents, fructose, rebaudioside A (stevia), and mixtures thereof are preferred.

Preferably, the thickening agent comprises a natural gum, more preferably gum arabic, xantham gum, and mixtures thereof.

Among flavoring agents, citric acid, aromas, and mixtures thereof are preferred.

Preferably, the composition according to the invention shows a pH from 4 to 6, more preferably from 4.2 to 5.8, even more preferably from 4.3 to 5.6, even more preferably from 4.4 to 5.4, even more preferably from 4.5 to 5.2.

Preferably, the composition according to the invention has a density of from 1.18-1.24 g/ml (determined measuring at 25 °C the ratio between the weight and the volume composition, for example 50 ml).

In a further aspect, the present invention also relates to a process for preparing the composition according to the invention, said process comprising the steps of:
- providing at least one fucoidan;
- adding and mixing at least one collagen hydrolysate in an amount wherein the weight ratio between the at least one collagen hydrolysate and the at least one fucoidan is from 85:1 to 15:1; and
- obtaining the composition.

The process according to the present invention allows obtaining, starting from the aforesaid starting products, the composition according to the invention, using a simple and reliable procedure.

Preferably, in the step of adding and mixing at least one collagen hydrolysate, the amount of the at least one collagen hydrolysate is such that the weight ratio between the at least one collagen hydrolysate and the at least one fucoidan is from 75:1 to 25:1, more preferably from 65:1 to 30:1, still more preferably from 45:1 to 35:1.

In a preferred embodiment, the process according to the present invention further comprises:
- adding and mixing one or more components selected from the group consisting of: water, preservative, nutrient, sweetening agent, thickening agent, flavoring agent, and mixtures thereof. Said one or more further components may be added all together or separately.

Preferably, after the step of adding and mixing the at least one collagen hydrolysate, the mixture thus obtained is maintained under stirring for a time interval of from 2 to 12 hours, more preferably from 4 to 10 hours, more preferably from 6 to 8 hours.

Preferably, the process according to the present invention further comprises:
- adjusting the pH to a value of from 4 to 6, more preferably from 4.2 to 5.8, even more preferably from 4.3 to 5.6, even more preferably from 4.4 to 5.4, even more preferably from 4.5 to 5.2.

Preferably, the process according to the present invention is carried out at a temperature from 10 °C to 65 °C, more preferably from 20 °C to 60 °C, more preferably from 22 °C to 60 °C.

In a preferred embodiment, the process according to the invention comprises the steps of:
a. providing at least one fucoidan;
b. adding to and mixing with the at least one fucoidan a first group of components selected from the group consisting of: water, preservative, nutrient, and mixtures thereof, to obtain a first mixture, wherein said step b is preferably carried out at a temperature from 10 °C to 35 °C, preferably from 20 °C to 30 °C, even more preferably from 22 °C to 27 °C;
c. adding to and mixing with the first mixture of step b. at least one collagen hydrolysate in an amount wherein the weight ratio between the at least one collagen hydrolysate and the at least one fucoidan is from 85:1 to 15:1, to obtain a second mixture;
d. maintaining the second mixture under stirring at a temperature from 10 °C to 35 °C, preferably from 20 °C to 30 °C, even more preferably from 22 °C to 27 °C, for a time interval of from 2 hours to 12 hours, more preferably from 4 hours to 10 hours, more preferably from 6 hours to 8 hours;
e. adding to and mixing with the second mixture after step d. a second group of components selected from the group consisting of: water, sweetening agent, thickening agent, flavoring agent, and mixtures thereof, to obtain a third mixture;
f. optionally adjusting the pH of the third mixture after step e. to a value of from 4 to 6, more preferably from 4.2 to 5.8, even more preferably from 4.3 to 5.6, even more preferably from 4.4 to 5.4, even more preferably from 4.5 to 5.2; and
g. obtaining the composition.

The Applicant has indeed noted that, thanks to the combination of this specific steps and process conditions, an intimate and optimal mixing between the at least one collagen hydrolisate and the at least one fucoidan may be obtained, further improving the effectiveness of the composition in providing beneficial effects on synovial joints.

In a further aspect, the present invention relates therefore also to a composition obtainable by means of the process according to the invention.

Preferably, step e. comprises: pre-mixing the second group of components at a temperature of from 10 °C to 65 °C for at least 1 hour before addition to and mixing with the second mixture after step d.

In a further aspect, the present invention relates also to the composition according to the invention for use in the treatment of a synovial joint disease or a synovial joint injury in a patient.

The Applicant has indeed noted that thanks to the association of collagen hydrolysate and fucoidan in a specific weight ratio, the composition according to the invention provides beneficial therapeutic effects on synovial joints in case of a synovial joint disease or a synovial joint injury in a patient, helping the articular cartilage to protect synovial joints and to facilitate the transmission of loads with a low frictional coefficient.

Preferably, the patient of the treatment according to the invention is a human being.

Preferably, the synovial joint of the treatment according to the invention is a shoulder, an elbow, an ankle, a hip, a wrist, or a knee joint.

Preferably, the treatment of a synovial joint disease or a synovial joint injury according to the invention includes the symptomatology of said synovial joint disease or synovial joint injury. More preferably, diminishing the symptomatology of said synovial joint disease or synovial joint injury comprises mitigating pain and sufferings linked to joints movements.

Preferably, the joint disease of the treatment according to the invention is arthritis, more preferably ostheoarthritis.

Preferably, the joint injury of the treatment according to the invention includes a cartilage abrasion or tearing.

Preferably, the treatment according to the invention includes administering the composition according to the invention to the patient for a period of at least 1 month, more preferably of at least 3 months, at a dose of 10 g/day of collagen hydrolysate and at a dose of 250 mg/day of fucoidan.

In a further aspect, the present invention relates to a dietary supplement made of the composition according to the invention.

The Applicant has indeed noted that the association of collagen hydrolysate and fucoidan provides also beneficial non-therapeutic effects to the cartilage metabolism, providing to the same nutrients that may preserve or improve its elasticity and resistance in a healthy subject.

Thanks to the association of collagen hydrolysate and fucoidan in a specific weight ratio, the dietary supplement according to the invention is indeed suitable to assist or improve the function of synovial joints during or after physical activity in a healthy subject, thus for example assisting the performance of athletes or sport lovers.

The dietary supplement according to the invention can be provided in any form which makes it suitable for final use, for example it can be in liquid, solid or gel form.

In a preferred embodiment, the dietary supplement according to the invention is in gel form.

Preferably, the dietary supplement according to the invention is prepared in the form of a capsule, granules, tablets, candies, tablets, pasta, gel or cream.

In a preferred embodiment, the present invention relates to the use of the dietary supplement according to the invention, for assisting or improving the function of synovial joints during or after physical activity in a healthy subject, more preferably in a human being.

Preferably, the use of the dietary supplement according to the invention comprises administering the composition according to the invention to the healthy subject in an amount of 10 g/day of collagen hydrolysate and in an amount of 250 mg/day of fucoidan.

Further features and advantages of the invention will appear more clearly from the following description of some preferred embodiments thereof, made hereinafter by way of a non-limiting example with reference to the following exemplary examples.

### EXPERIMENTAL PART

### Example 1 - preparation of a composition comprising a collagen hydrolysate and a fucoidan with a weight ratio between the collagen hydrolysate and the fucoidan of 40:1

33.3 parts by weight of water, 0.1 parts by weight of potassium sorbate (granular product, purity 100%), 0.1 parts by weight of sodium benzoate (granular product, purity 99.9%), 1 part by weight of fucoidan (Fucus vesiculosus glyceric extract, Maritech^{®}), 0.3 parts by weight of zinc gluconate and 0.3 parts by weight of ascorbic acid were fed to a steel mixing vessel and mixed at 25 °C until a clean solution was obtained. Thus, 40 parts by weight of collagen hydrolysate (Fortigel^{™} by Gelita) were added and the mixture was kept under stirring overnight (7 hours) and subsequently left without stirring for 24 hours, thus obtaining a first mixture.

8.3 parts by weight of water, 10.5 parts by weight of fructose, 0.05 parts by weight of xantham gum and 0.15 parts by weight of gum Arabic were fed to a second mixing vessel and mixed at 55 °C for 3 hours, and then cooled to room temperature, thus obtaining a second mixture.

2 parts by weight of water, 0.05 parts by weight of rebaudioside A, 0.1 parts by weight of monohydrated citric acid and 0.4 parts by weight of lemon aroma were fed to a third mixing vessel and mixed at 25 °C until a clean solution was obtained thus obtaining a third mixture.

Thus, the second and third mixture were added to the first mixture under mixing at 25 °C , adjusting the pH to a value of 5.0, thus obtaining a final composition comprising a collagen hydrolysate and a fucoidan with a weight ratio between the collagen hydrolysate and the fucoidan of 40:1.

### Example 2 - evaluation of the composition for diminishing the symptomatology of osteoarthritis of knee joint, compared with intraarticular hyaluronic acid or platelet-rich plasma

The efficacy of the composition according to Example 1 ("Ex1") to reduce the symptomatology of osteoarthritis of knee joint, compared with intraarticular infiltrations of hyaluronic acid ("HA", Durolane^{®}; Bioventus LLC, Durham, NC) and platelet-rich plasma ("PRP", autologous process made by the blood of the patient) was tested and verified by means of a double blind clinical trial.

108 patients selected with the criteria summarized in TABLE 1 herebelow were randomly assigned in 3 groups, respectively Group 1, Group 2 and Group 3, each of 36 patients.

**TABLE 1**

| Inclusion criteria | Exclusion criteria | Elimination criteria |
|---|---|---|
| Accept contentment | Pregnant women | Not coming to check up |
| Osteoarthrosis diagnose by radiological and clinical criteria | Breastfeeding women | Stop treatment |
| II-III Kellgren- Lawrence score | Previous knee surgery | |
| Both genders | Arthroscopy with at least 1 year before | |
| Between 40 and 90 years old | Intraarticular injections (hyaluronic acid and platelet- rich plasma) 9 months before | |
| | Use of glucosamine and chondroitin 6 months before | |
| | Use herbology products | |
| | Comorbidities like: gout, heart failure, diabetes mellitus (HbA1c> 7%, ADA) and hypertension (>140/90 mm Hg, AHA) | |

The duration of the clinical trial was of 6 months, with the patient scheduled every 14 days with surveys on VAS (visual analogue scale), and WOMAC (Western Ontario and McMaster Universities Osteoarthritis Index) at the beginning of the trial (baseline), at 1 month, at 3 months from said beginning, and at the end of 6 months. A blood sample was taken to all patients.

All of the patients received an intraarticular injection and an oral therapy (see Table 2).

**TABLE 2**

| | | |
|---|---|---|
| | intraarticular injection | oral therapy |
| Group 1 | placebo (one shot administration of 3 ml of saline solution at the beginning of the trial) | Composition according to Example 1 (23 grams per day) |
| Group 2 | HA (one shot administration of 3 ml at the beginning of the trial) | placebo (liquid chlorophyll) (23 grams per day) |
| Group 3 | PRP (one shot administration of 3 ml at the beginning of the trial) | placebo (liquid chlorophyll) (23 grams per day) |

In the clinical trial, a VAS scale was used for measuring the patients subjective pain, in which 0 corresponded to "no pain" and 10 to "unbearable pain" according to the common practice as reported, for example in https://www.physio-pedia.com/Visual_Analogue_Scale.

As to the WOMAC scale, it was used in the clinical trial to measure five items for pain (score range 0-20), two for stiffness (score range 0-8) and 17 for functional limitation (score range 0-68), according to the common practice as reported, for example in Bonnie B. et al. "Longitudinal comparison of the Health Assessment Questionnaire (HAQ) and the Western Ontario and McMaster Universities Osteoarthritis Index (WOMAC)", Arthritis Care & Research, Volume 51, Issue 5, 730-737 (https://onlinelibrary.wiley.com/doi/10.1002/art.20695).

In TABLE 3 the data obtained at the beninning ("baseline") and after 1 and 3 months from the beginning of the clinical trial.

**TABLE 3**

| | VAS | | | WOMAC | | |
|---|---|---|---|---|---|---|
| | baseline | 1 month | 3 months | baseline | 1 month | 3 months |
| Ex1 | 6.83 | 4 | 2.33 | 50.66 | 21 | 19.83 |
| HA | 8.6 | 6.4 | 5.7 | 44.1 | 27.7 | 26.6 |
| PRP | 8 | 5 | 5.5 | 54 | 26 | 30.5 |

It was noted that in the group of patients treated with the composition according to Example 1 (Ex1), the VAS score diminished 4.5 points (about 66% of the baseline value) from the baseline to the third treatment month. In the WOMAC scale, the score diminished 30.83 points (about 61% of the baseline value) from the baseline to the third treatment month.

It was also noted that in the group of patients treated with hyaluronic acid (HA), the VAS score diminished only of 2.9 points (about 34% of the baseline value) from the baseline to the third treatment month and in the WOMAC scale, the score diminished of 17.75 points, corresponding to a 40% reduction of the baseline value.

Finally, it was noted that in the group of patients treated with Platelet-rich plasma (PRP), the VAS score diminished only of 2.5 points (about 31% of the baseline value) from the baseline to the third treatment month whereas in the WOMAC scale, the score diminished of 23.5 points, corresponding to a 40% reduction of the baseline value in the third treatment month. It was also noted that the WOMAC value of this group of patient was the only one with a third month value (30.5) worse than the first month value (26), that might indicate a loss of efficacy of the treatment.

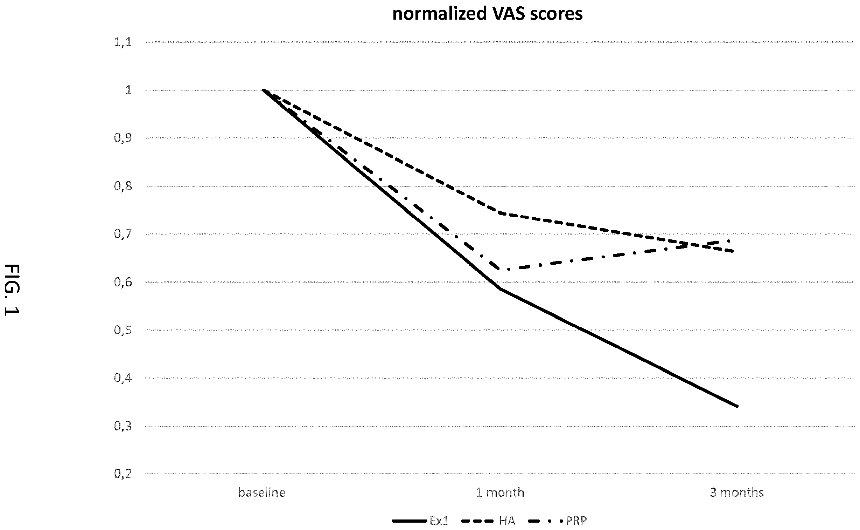

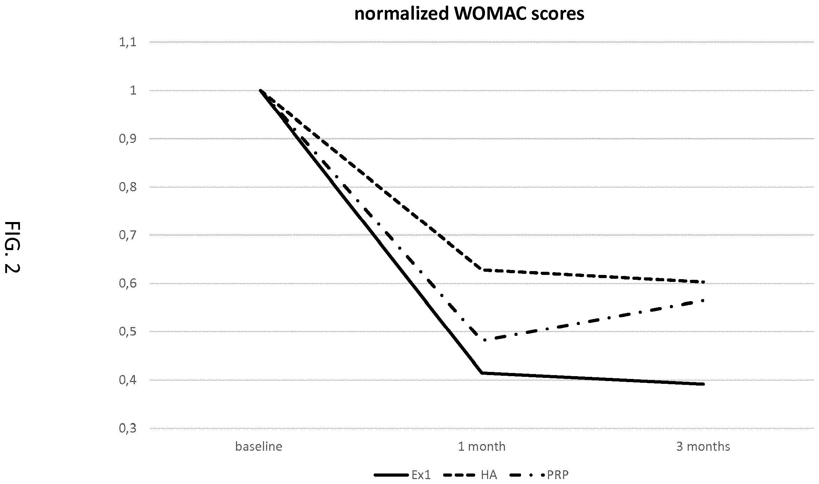

Figure 1 and 2 show the data obtained at the end of the clinical trial respectively for the VAS and WOMAC scores, reproduced on a normalized scale.

From the data obtained, it was possible to note that the treatment with the composition according to Example 1 resulted in a remarkably stronger improvement in the conditions of the patients.

## Claims

1. A composition comprising at least one collagen hydrolysate and at least one fucoidan, wherein the weight ratio between the at least one collagen hydrolysate and the at least one fucoidan is from 85:1 to 15:1.

2. The composition according to claim 1, wherein the at least one collagen hydrolysate has an average molecular weight in the range of 2,000-5,000 daltons.

3. The composition according to claim 1 or 2, wherein the at least one fucoidan is a Fucus vesiculosus extract.

4. A process for preparing the composition according to any one of claims 1-3, said process comprising the steps of:
- providing at least one fucoidan;
- adding and mixing at least one collagen hydrolysate in an amount wherein the weight ratio between the at least one collagen hydrolysate and the at least one fucoidan is from 85:1 to 15:1; and
- obtaining the composition.

5. The process according to claim 4, comprising the steps of:
a. providing at least one fucoidan;
b. adding to and mixing with the at least one fucoidan a first group of components selected from the group consisting of: water, preservative, nutrient, and mixtures thereof, to obtain a first mixture, wherein said step b is preferably carried out at a temperature from 10 °C to 35 °C;
c. adding to and mixing with the first mixture of step b. at least one collagen hydrolysate in an amount wherein the weight ratio between the at least one collagen hydrolysate and the at least one fucoidan is from 85:1 to 15:1, to obtain a second mixture;
d. maintaining the second mixture under stirring at a temperature from 10 °C to 35 °C for a time interval of from 2 hours to 12 hours;
e. adding to and mixing with the second mixture after step d. a second group of components selected from the group consisting of: water, sweetening agent, thickening agent, flavoring agent, and mixtures thereof, to obtain a third mixture;
f. optionally adjusting the pH of the third mixture after step e. to a value of from 4 to 6; and
g. obtaining the composition.

6. A composition obtainable by means of the process according to claim 4 or 5.

7. A composition according to any one of claims 1-3 or 6 for use in the treatment of a synovial joint disease or a synovial joint injury in a patient.

8. The composition for use according to claim 7, wherein the joint disease is arthritis.

9. The composition for use according to claim 8, wherein the arthritis is ostheoarthritis.

10. The composition for use according to claim 7, wherein the joint injury includes a cartilage abrasion or tearing.

11. The composition for use according to any one of claims 7-10, wherein the composition is administered to the patient for a period of at least 1 month at a dose of 10 g/day of collagen hydrolysate and at a dose of 250 mg/day of fucoidan.

12. A dietary supplement made of the composition according to any one of claims 1-3 or 6.

13. The dietary supplement according to claim 12, in the form of capsule, granulate, pill, candy, tablet, paste, gel, or cream.

14. A use of the dietary supplement according to claim 12 or 13, for assisting or improving the function of synovial joints during or after physical activity in a healthy subject.

## Patentansprüche

1. Zusammensetzung, umfassend mindestens ein Kollagenhydrolysat und mindestens ein Fucoidan, wobei das Gewichtsverhältnis zwischen dem mindestens einen Kollagenhydrolysat und dem mindestens einen Fucoidan 85:1 bis 15:1 beträgt.

2. Zusammensetzung nach Anspruch 1, wobei das mindestens eine Kollagenhydrolysat ein durchschnittliches Molekulargewicht im Bereich von 2.000 - 5.000 Dalton aufweist.

3. Zusammensetzung nach Anspruch 1 oder 2, wobei das mindestens eine Fucoidan ein Fucus vesiculosus-Extrakt ist.

4. Verfahren zur Herstellung der Zusammensetzung nach einem der Ansprüche 1 bis 3, wobei das Verfahren die folgenden Schritte umfasst:
- Bereitstellen mindestens eines Fucoidans;
- Zugeben und Mischen mindestens eines Kollagenhydrolysats in einer Menge, bei der das Gewichtsverhältnis zwischen dem mindestens einen Kollagenhydrolysat und dem mindestens einen Fucoidan 85:1 bis 15:1 beträgt; und
- Gewinnen der Zusammensetzung.

5. Verfahren nach Anspruch 4, das die folgenden Schritte umfasst:
a. Bereitstellen mindestens eines Fucoidans;
b. Zugeben und Mischen einer ersten Gruppe von Bestandteilen, ausgewählt aus der Gruppe bestehend aus: Wasser, Konservierungsmittel, Nährstoff und Mischungen davon, mit dem mindestens einen Fucoidan, um eine erste Mischung zu erhalten, wobei der Schritt b vorzugsweise bei einer Temperatur von 10 °C bis 35 °C durchgeführt wird;
c. Zugeben und Mischen von mindestens einem Kollagenhydrolysat in einer Menge, mit der ersten Mischung aus Schritt b., wobei das Gewichtsverhältnis zwischen dem mindestens einen Kollagenhydrolysat und dem mindestens einen Fucoidan 85:1 bis 15:1 beträgt, um eine zweite Mischung zu erhalten;
d. Halten der zweiten Mischung unter Rühren bei einer Temperatur von 10 °C bis 35 °C während eines Zeitintervalls von 2 Stunden bis 12 Stunden;
e. Zugeben und Mischen einer zweiten Gruppe von Komponenten, ausgewählt aus der Gruppe bestehend aus: Wasser, Süßstoff, Verdickungsmittel, Geschmacksstoff und Mischungen davon, mit der zweiten Mischung nach Schritt d., um eine dritte Mischung zu erhalten;
f. gegebenenfalls Einstellen des pH-Werts des dritten Gemischs nach Schritt e. auf einen Wert von 4 bis 6; und
g. Gewinnen der Zusammensetzung.

6. Zusammensetzung, erhältlich durch das Verfahren nach Anspruch 4 oder 5.

7. Zusammensetzung nach einem der Ansprüche 1 bis 3 oder 6 zur Verwendung bei der Behandlung einer Erkrankung des Synovialgelenks oder einer Verletzung des Synovialgelenks bei einem Patienten.

8. Die Zusammensetzung zur Verwendung nach Anspruch 7, wobei die Gelenkerkrankung Arthritis ist.

9. Zusammensetzung zur Verwendung nach Anspruch 8, wobei die Arthritis Ostheoarthritis ist.

10. Zusammensetzung zur Verwendung nach Anspruch 7, wobei die Gelenkverletzung eine Knorpelabnutzung oder einen Knorpelriss umfasst.

11. Zusammensetzung zur Verwendung nach einem der Ansprüche 7 bis 10, wobei die Zusammensetzung dem Patienten über einen Zeitraum von mindestens 1 Monat in einer Dosis von 10 g/Tag Kollagenhydrolysat und in einer Dosis von 250 mg/Tag Fucoidan verabreicht wird.

12. Nahrungsergänzungsmittel, hergestellt aus der Zusammensetzung nach einem der Ansprüche 1 bis 3 oder 6.

13. Nahrungsergänzungsmittel nach Anspruch 12 in Form von Kapsel, Granulat, Pille, Bonbon, Tablette, Paste, Gel oder Creme.

14. Verwendung des Nahrungsergänzungsmittels nach Anspruch 12 oder 13 zur Unterstützung oder Verbesserung der Funktion der Synovialgelenke während oder nach körperlicher Aktivität bei einem gesunden Menschen.

## Revendications

1. Composition comprenant au moins un hydrolysat de collagène et au moins un fucoïdane, où le rapport pondéral entre l'au moins un hydrolysat de collagène et l'au moins un fucoïdane est de 85:1 à 15:1.

2. Composition selon la revendication 1, où l'au moins un hydrolysat de collagène a un poids moléculaire moyen allant de 2 000 à 5 000 daltons.

3. Composition selon la revendication 1 ou 2, où l'au moins un fucoïdane est un extrait de Fucus vesiculeux.

4. Procédé de préparation de la composition selon l'une quelconque des revendications 1 à 3, ledit procédé comprenant les étapes suivantes :
- la fourniture d'au moins un fucoïdane ;
- l'ajout et le mélange d'au moins un hydrolysat de collagène en une quantité où le rapport pondéral entre l'au moins un hydrolysat de collagène et l'au moins un fucoïdane est de 85:1 à 15:1 ; et
- l'obtention de la composition.

5. Procédé selon la revendication 4, comprenant les étapes suivantes :
a. la fourniture d'au moins un fucoïdane ;
b. l'ajout et le mélange à l'au moins un fucoïdane d'un premier groupe de composants choisis dans le groupe constitué par : l'eau, un conservateur, un nutriment, et leurs mélanges, pour obtenir un premier mélange, où ladite étape b est de préférence réalisée à une température de 10°C à 35°C ;
c. l'ajout et le mélange au premier mélange de l'étape b. d'au moins un hydrolysat de collagène en une quantité où le rapport pondéral entre l'au moins un hydrolysat de collagène et l'au moins un fucoïdane est de 85:1 à 15:1, pour obtenir un deuxième mélange ;
d. le maintien du deuxième mélange sous agitation à une température de 10°C à 35°C pendant un intervalle de temps de 2 heures à 12 heures ;
e. l'ajout et le mélange au deuxième mélange après l'étape d. d'un second groupe de composants choisis dans le groupe constitué par : l'eau, un agent édulcorant, un agent épaississant, un agent aromatisant, et leurs mélanges, pour obtenir un troisième mélange ;
f. éventuellement, l'ajustement du pH du troisième mélange après l'étape e. à une valeur comprise entre 4 et 6 ; et
g. l'obtention de la composition.

6. Composition pouvant être obtenue au moyen du procédé selon la revendication 4 ou 5.

7. Composition selon l'une quelconque des revendications 1 à 3 ou 6 pour une utilisation dans le traitement d'une maladie de l'articulation synoviale ou d'une lésion de l'articulation synoviale chez un patient.

8. Composition à utiliser selon la revendication 7, où la maladie de l'articulation est l'arthrite.

9. Composition à utiliser selon la revendication 8, où l'arthrite est l'arthrose.

10. Composition à utiliser selon la revendication 7, où la lésion articulaire comprend une abrasion ou une déchirure du cartilage.

11. Composition à utiliser selon l'une quelconque des revendications 7 à 10, où la composition est administrée au patient pendant une période d'au moins 1 mois à une dose de 10 g/jour d'hydrolysat de collagène et à une dose de 250 mg/jour de fucoïdane.

12. Complément alimentaire constitué de la composition selon l'une quelconque des revendications 1 à 3 ou 6.

13. Complément alimentaire selon la revendication 12, sous forme de gélule, granulé, pilule, bonbon, comprimé, pâte, gel ou crème.

14. Utilisation du complément alimentaire selon la revendication 12 ou 13, pour aider ou améliorer la fonction des articulations synoviales pendant ou après une activité physique chez un sujet sain.
